# EUROPEAN PATENT SPECIFICATION

(11) **EP 1 307 227 B1**
(45) Date of publication and mention of the grant of the patent: **02.11.2005**
(21) Application number: 01971777.6
(22) Date of filing: 24.07.2001
(51) Int. Cl.: A61K 41/00, A61P 31/00

(54) **PHARMACEUTICAL COMPOSITIONS**
PHARMAZEUTISCHE FORMULIERUNGEN
COMPOSITIONS PHARMACEUTIQUES

(30) Priority: 04.08.2000 GB 0019283
(43) Date of publication of application: 07.05.2003
(73) Proprietor: Novartis AG, 4056 Basel (CH); Novartis Pharma GmbH, 1235 Vienna (AT)
(72) Inventor: BRUNO-RAIMONDI, Alfredo, Emilio, CH-4105 Biel-Benken (CH)
(74) Representative: Gros, Florent
(86) International application number: PCT/EP2001/008558
(87) International publication number: WO 2002/011764

(56) References cited:
- EP-A- 0 503 988
- WO-A-99/04628
- DE-A- 10 017 996
- US-A- 5 947 956
- RISPLER J: "ONYCHOMYCOSIS TREATED WITH CARBON DIOXIDE LASER NAIL ETCHING." SIXTH ANNUAL MEETING OF THE AMERICAN SOCIETY FOR LASER MEDICINE AND SURGERY, BOSTON, MASS., USA, MAY 24-26, 1986. LASERS SURG MED. (1986) 6 (2), 251. , XP001053492

## Description

The present invention relates to a method for the topical treatment of nail diseases, e.g. onychomycosis, after preparation of the infected nail with e.g. a pulsed laser beam.

Diseases of the nail, such as onychomycosis, atopic eczema, or psoriasis of the nail are difficult to treat. Although for some of these diseases effective treatment by the systemic, e.g. oral, route is available, there is still the need for an effective topical method of treatment.

Onychomycosis accounts for up to 50% of all nail diseases and affects 2% to 18% or more of the world's population. Some studies suggest that up to 48% of the population may be affected by age 70. Toenail infection is several times more common than fingernail infection and is more difficult to treat because of its slower growth rate.

Terbinafine is an orally effective anti-fungal agent, available under the registered trademark Lamisil. It is effective in a wide range of fungal infections. Terbinafine is particularly useful against dermatophytes, contagious fungi that invade dead tissues of the skin or its appendages such as stratum comeum, nails, and hair. The effects of these fungi on the nails may be unsightly, seriously complicate foot-care, have a deleterious impact on patients' overall quality of life, and well-being and impair the patients' ability to work. If left untreated, the fungi can deform toe-nails permanently and lead to pain on walking. Additionally the fungi can lead to fissures in the skin encouraging bacterial infections. Serious complications as a result of these infections may occur in people suffering from diabetes such as diabetic foot syndrome including primary disease-related complications, e.g. gangrene, that, ultimately, can be life-threatening or require amputations. Other high-risk patient sub-groups include patients infected with human immunodeficiency virus (HIV), patients with acquired immunodeficiency syndrome (AIDS), and patients with other types of immunosuppression (e.g. transplant recipients and patients on long-term corticosteroid therapy). Diagnosis is confirmed by demonstrating the pathogenic fungus in scrapings of the lesions either by microscopic examination or by culture.

For the onychomycosis use, an antifungal, e.g. terbinafine, is normally orally administered as an immediate release tablet. Terbinafine treatment is typically required over 12 weeks. The progress of its clinical effectiveness is seen with growth of the healthy nail, pushing out and replacing, the diseased unsightly nail containing debris and dead fungus. About 10 months is taken for a totally new toe-nail to form.

Whereas e.g. terbinafine is highly active upon oral administration, systemic treatment of onychomycosis offers some disadvantages, e.g. exposure of the whole organism to the drug substance and the need for rather high doses. Therefore the possibility of local, namely topical treatment is highly desirable and would be preferred by many patients. However, one difficulty in the topical treatment of onychomycosis or other nail diseases may be related to insufficient penetration of the drugs into deeper layers of the nail and nail bed.

The nail plate is formed by layers of keratinised cells produced by the nail matrix, a highly proliferative epidermal tissue. Besides keratin, the rigid structure of the nail contains numerous trace minerals including calcium. The nail plate overlays the nail bed, a noncomified tissue. At the interface, nail bed cells are carried distally by the nail plate during the growth towards the free margin. The keratinisation of the nail plate in the matrix occurs both on the dorsal and ventral side of the forming nail plate. There are at least two discernible macroscopic strata, with possible a third. These are the dorsal nail plate and the intermediate nail plate with the third under-layer or ventral plate contributed by the cells of the lunula. The dorsal plate is harder laminated thus more compact and making the penetration of compounds more difficult.

The hydrated nail behaves as a hydrogel of high ionic strength forming a thick hydrophilic barrier making it extremely difficult for hydrophobic drugs to penetrate the nail plate down to the nail bed. The thickness of the nail and its relatively compact structure make it even more difficult for topically applied drugs, e.g. antifungal agents, to penetrate the nail.

According to the present invention it has now been found that onychomycosis can be successfully treated by forming one or more small orifices, e.g. traversing the entire nail plate or etching the nail plate, and administering e.g. an antifungal-, e.g. a terbinafine-, containing composition to the nail. Preferably, the orifices may have a size (diameter) of e.g. 10 µm (microns) to 2 mm, e.g. 50 µm (microns) to 1 mm, e.g. 140 µm (microns) to 1 mm.

Accordingly, in one aspect the present invention provides a method for the treatment of onychomycosis which method comprises forming one or more orifices in the nail and administering e.g. an antifungal-containing, e.g. preferably a terbinafine-, containing, pharmaceutical composition to the nail.

"Orifice" as herein described means any small hole or depression that penetrates 80 to 100% of the nail plate, preferably 90 to 99%.

To date, orifices in finger- or toenails have been produced with a mechanical drill, e.g. as described in US 4,180,058, or with a heated wire for burning a hole. In US 5,947,956, a laser apparatus for making holes and etchings is described.

As antifungal, allyl amines such as terbinafine or naftifine, benzylamines such as butenafine, and/or azole-based antifungals such as tioconazole, econazole, oxiconazole, itroconazole, fluconazole, ketoconazole, miconazole and clotrimazole may be used. Allyl amines and benzyl amines may be in free base form or acid addition salt form, preferably in form of hydrochloride salt.

The antifungal-containing pharmaceutical composition may comprise a highly concentrated antifungal formulation from about 10 to about 100% of antifungal by weight of the composition, e.g. more than about 70%, or e.g. about 100%, e.g. substantially pure antifungal, e.g. terbinafine, powder.

Alternatively, the antifungal- containing pharmaceutical composition comprises antifungal from about 1 to about 10% by weight of the composition.

The antifungal-containing pharmaceutical composition may be e.g. liquid, viscous or semi-solid, e.g. in form of a cream, a gel, a solution, a lotion, an ointment, a patch or a nail varnish; the formulation may e.g. be a liposomal preparation. If the composition is viscous, it may be warmed up before pouring it in the orifice, e.g. to facilitate penetration. Alternatively, the antifungal-containing pharmaceutical composition may be e.g. solid, e.g. a powder.

The preferred type and strength of formulation may be chosen according to e.g. the degree of infection of nail and/or size of holes. Furthermore, a surfactant may be added to the antifungal formulation, e.g. to facilitate its penetration into and through the orifices.

In another aspect, the present invention provides for the use of an antifungal, e.g. terbinafine, to produce a medicament to be administered to penetrate effectively an orifice of a nail.

Before administering the antifungal-containing pharmaceutical composition, the nail, e.g. after being prepared with the orifices, may be treated with a surfactant e.g. such as i) natural products, e.g. Aloe Vera, e.g. in form of a gel, or ii) a non-ionic surfactant which is inert, non-irritant, and does not have a pharmaceutical response, e.g. in order to facilitate penetration of the anti-fungal formulation.

Immediately after administering the antifungal-, e.g. terbinafine-, containing pharmaceutical composition to the nail, a, e.g. protective, e.g. inert, layer comprising e.g. a nail varnish, porcelain layer, an artificial nail or a polymer foil, e.g. a patch, may be applied onto the nail. This may ensure that the pharmaceutical composition remains in the nail and may also prevent bacteria and dirt reaching the nail bed. The protective layer preferably also comprises an anti-fungal. Application of e.g. a colored nail varnish may mask the unpleasant appearance of the infected nail.

In a further aspect the present invention provides a method as described above which method further comprises administering a, e.g. protective, layer onto the nail.

As the nail grows, the small orifice typically closes, thus trapping the antifungal into the nail plate. Accordingly, after some time, e.g. a few days to one week, application of a protective layer may no longer be needed.

The present applicants have found that the orifice in the nail may be easily formed in a fraction of a second by a device which comprises a laser, e.g. an Erbium (Er:YAG) laser, Neodym (Nd:YAG) laser, OPO laser, Holmium (Ho:YAG), a nitrogen laser or CO₂ laser where YAG stands for yitrium aluminium garnet.

In another aspect, the present invention provides a method for the treatment of a nail infected with onychomycosis which method comprises forming one or more orifices in the nall with a device comprising a laser, e.g. an Erbium (Er:YAG) laser, Holmium (Ho:YAG) or nitrogen laser, and administering an antifungal-containing pharmaceutical composition to the nail.

The use of a laser-based device to form an orifice in the nail according to the present invention is especially advantageous because of e.g. high precision, high speed, lack of or little pain, and no risk of bleeding or of secondary infections.

Laser surgery, i.e. the method of the present invention to perforate the nail, is based on the photo-ablation process which refers to the melting and explosion of hard tissues. Pulsed laser irradiation of a selected wavelength, power and pulse duration according to the thermal, mechanical and spectral characteristics of the tissue of interest, achieve photo-ablation. The irradiation of a tissue with a pulsed laser of low to moderate power density induces thermal changes through absorption with minor mechanical and reversible changes. However, at much higher power and when the confinement of a given energy in a defined volume during a short time satisfies the threshold conditions, hard tissue can be selectively destructed, e.g. for drilling or cutting purposes. The deposited electromagnetic energy is almost entirely transformed into mechanical energy (i.e. hv ≈ mv²/2) and the illuminated region is ejected in the form of debris escaping the orifice at ca. 1'000 m/s. In a "clean", i.e. efficient, photoablation process, as the debris removes most of the deposited energy, the irradiated tissue, e.g. the tissue in contact with the nail bed, is not heated thus reducing the causes for pain.

Although the nail bed is known to be very sensitive, according to the present invention it has been shown that nail diseases can be treated using a laser based technology with minimal patient discomfort. It has been found that the associated pain is minimal and can be tolerated without anaesthetics.

As the ablation temperature is e.g. higher than about 100°C, disinfection of nail and/or nail orifices may not be required. Therefore, the present invention provides a method as described above which method does not involve a disinfection step.

Because the spectral characteristics of human tissues in the NIR region are dominated by the spectrum of water, the lasers that can be used for tissue micromachining are the CO₂ gas laser lasing at λ = 10.6 µm, and the two most recently developed solid state lasers, the Ho:YAG and Er:YAG delivering light (electromagnetic radiation) at λ = 2.1 µm and λ = 2.94 µm, respectively. A Nd:YAG laser or an OPO laser may also be used. According to the present invention, the Ho:YAG or Er:YAG laser are preferred.

According to the present invention it has been found that one or more small orifices may be formed with a, preferably tightly focussed, single laser shot of only less than ca. 250 mJ of power, e.g. 50 mJ, ca. 250 µs of duration at a repetition rate of 3 Hz for a healthy nail of about 0.7 mm thickness. Although we do not want to be bound by any theory, it is believed that the surprising much easier to achieve photoablation conditions in the nail, as compared to bone or teeth, appears to berelated to its structure. As mentioned above, the nail behaves as a hydrogel. Consequently its spectrum is dominated by the absorption of water and, the melting point of humidified keratine should be at a much lower temperature than that of hydroxilapatite or enamel contained in bone or teeth, respectively. For this reason, typically the orifices are substantially circular, conical, frusto-conical, hemi-spherical or cylindrical in cross-section, preferably cylindrical, mimicking the stark focussed laser beam profile. As the process occurs faster than the thermal diffusion rate in nail, no damage in the orifices in the form of craters are observed. Most importantly, the orifices are done with a single shot or a few weak laser shots thus reducing the heating of the nail, a source of pain, to a minimum. This aspect is also time-relevant if one wishes to make arrays of orifices as explained below. With a more refined optical set-up, using e.g. masks, the orifices may be made much smaller or, with the use of Diffractive Optical Elements (DOEs), such as a Dammann grating arrays of orifices can be done simultaneously.

According to the present invention, the nail can be used as a natural depot, e.g. reservoir, wherein an array of equally spaced laser formed orifices in the nail is made and subsequently filled with the antifungal-containing composition to be released, e.g. slowly released, into the nail bed. Because it takes the nail 5 to 10 months to regenerate, the orifices constitute an ideal depot, e.g. reservoir, for the slow delivery of antifungal, e.g. terbinafine, into the nail bed for the topical treatment of onychomycosis.

The laser-based device, e.g. laser light delivery device, for forming one or more orifices in one or more nails may comprise the following elements:
(a) a laser capable of inducing ablation on the nail plate, i.e. for perforating nail, e.g. making orifices, e.g. as main photoablation laser, e.g. a pulsed, e.g. solid state, laser light source, e.g. Erbium (Er:YAG) laser (λ = 2.94 microns), a Nd:YAG laser, an OPO laser, a Ho:YAG laser (λ = 2.1 microns), or a CO₂ laser (λ = 10.6 microns), preferably an Erbium (Er:YAG) or Holmiun (Ho:YAG) laser
(b) supporting means to fix/position, e.g. by a clamp, the toe or finger to be treated, leaving the nail plate uncovered for the laser illumination or exposure,
(c) preferably a second visible continuous wave (cw) laser, a so-called pointing or targeting laser (acting as a pointer) of e.g. low power, e.g. a laser emitting in the visible region, e.g. a red He-Ne lasing at λ = 633 rim, or e.g. a laser diode, may be used to ensure a higher micromachining precision, i.e. visualisation of the spots to be perforated,
(d) a computer controlled xyz translation stage module to position the laser beam in the desired area of the nail preferably a so-called laser beam scanner. Alternatively the support
(b) may be mounted on this translation stage so that the laser beam is fixed and the toe or finger moves. Alternatively, the toe or finger may be fixed and the laser beam is mounted on the translation stage to move on the nail plate.
(e) means for directing laser beams, e.g. a mirror, e.g. a dichroic mirror, or a prism, to coaxially mix the laser beams from the ablation laser (a) and optionally the pointing laser (d),
(f) means to remove the nail debris, e.g. a nail debris removing disk, e.g. vacuum system or a flat thin piece of, e.g. round, material which is transparent to the laser beam wavelengths, e.g. a round disk made of quartz, e.g. fused silica, may be used to prevent dirtying of the focussing optics elements (i),
(g) means to clean the nail debris removing disk (f) from the nail debris, e.g. a thin tube ejecting a jet of sterilized water,
(h) means to ensure that the nail debris removing disk (f) is clean when the ablation laser is fired, e.g. a wiper, e.g. made of a flexible material, e.g. rubber or silicon polymer, or a brush,
(i) one or more focussing optics elements, e.g. manual or in an auto-focus mode, comprising one or more lenses placed between the lasers (a) and (d) and the nail debris removing disk (f),
(j) computing means, e.g. a personal computer, serving e.g. the following purposes:
   to monitor the nail plate by means of a video camera or charged coupled device camera (k),
   to place the laser beams (a) and (d) to the points of the nail plate where orifices are to be formed by means of a computer controlled xyz translation stage (c), to control and/or select the different laser parameters, e.g. the firing of the laser when the desired position of the xyz
   translation stage (c) has been reached, or the laser power, the pulse duration, or wavelength, e.g. if the laser is a tunable laser,
(k) means for monitoring the nail plate, e.g. a video camera or charged coupled device camera, to monitor the nail plate on the screen of computing means, e.g. a personal computer (j), preferably a charged coupled device camera,
(I) feedback means, e.g. a sensor, e.g. a photoacoustic sensor made of a piezoelectric (PZE) material, to ensure that the laser stops after the orifice has reached a predetermined depth, e.g. the nail bed. Preferably the ablation laser (a) conditions are chosen to form an orifice with a single shot without reaching soft tissue. The feedback sensor may be used to choose and optimize this condition starting from the first orifice formed,
(m) an optical element may be used to multiplex the laser beams (a) and (d), e.g. a diffractive optical element, e.g. a Dammann grating, to make more than one orifice, e.g. an array of equally spaced orifices, by a single shot thereby avoiding to make the orifices one-by-one in a subsequent mode.

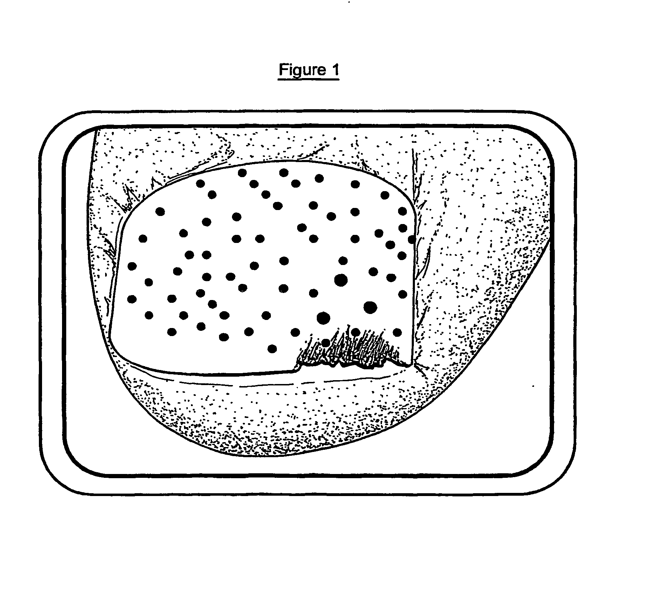

Preferably, the operator uses a software that makes use of the image of the captured and stored nail image to help the operator define the pattern, size, geometry etc. of the orifice-array and, with this information, calculate the individiual orifice coordinates with respect to the nail, compute corresponding laser ablation conditions, and/or run the positioning elements, e.g. run the laser or lasers and the hardware. Figure 1 shows an example of an orifice array.

Preferably, the laser light delivery device, e.g. especially the support (b), the mirror (e) and the device to remove the nail debris (f), is easy to clean and may be run under sterile conditions.

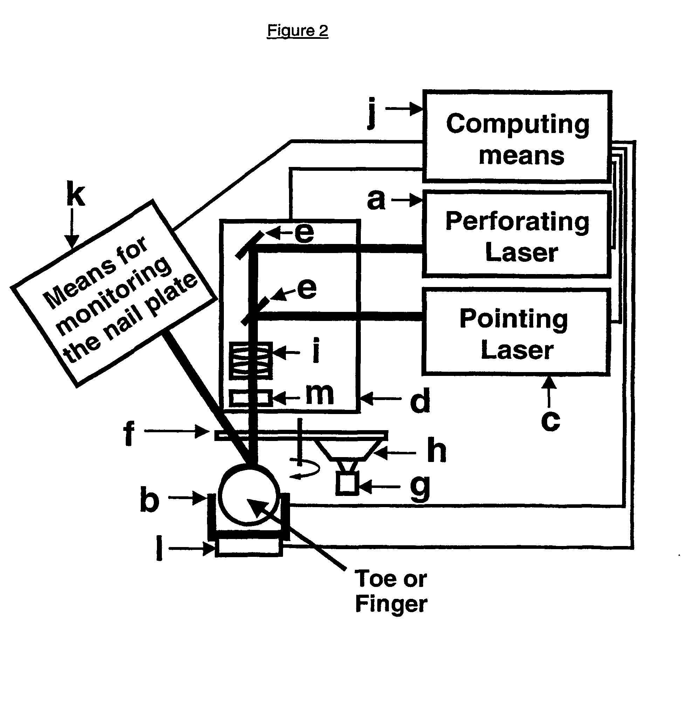

Accordingly, in a further aspect the present invention provides a device, e.g. for the use in a method according to the present invention, e.g. as described in Figure 2, which device comprises
(a) a laser for perforating nail and optionally one or more of the following elements
(b) supporting means to fix/position the toe or finger to be treated,
(c) a pointing laser,
(d) a xyz translation stage moduie,
(e) means for directing laser beams,
(f) means to remove the nail debris,
(g) means to clean the nail debris removing disk (f) from the nail debris,
(h) means to ensure that the nail debris removing disk (f) is clean when the ablation laser is fired,
(i) one or more focussing optics comprising one or more lenses,
(j) computing means,
(k) means for monitoring the nail plate (I) feedback means,
(m) a diffractive optical element,
(n) software to define the pattern, size, geometry etc. of the orifice-array, to calculate the individiual orifice coordinates, to compute corresponding laser ablation conditions, and/or to run the positioning elements.

Preferably, the device, e.g. for the use in a method according to the present invention comprises the following elements:
(a) a laser for perforating nail, e.g. for making orifices, e.g. a solid state pulsed laser light source, e.g. Erbium (Er:YAG) laser, Neodym (Nd:YAG) laser, OPO laser, Holmiun (Ho:YAG), or CO₂ laser, preferably Erbium (Er:YAG) laser, Holmiun (Ho:YAG), or CO₂ laser, even more preferably an Erbium (Er:YAG) laser, e.g. as main photoablation laser,
(b) supporting means to fix/position the toe or finger to be treated,
(c) a pointing laser, e.g. a visible continuous wave (cw) laser of e.g. low power, from e.g. a laser emitting in the visible region, e.g. a red He-Ne laser or a laser diode, and
(d) optionally a xyz translation stage module.

The following Examples illustrate the invention.

### Example 1

A series of orifices (ca. 20 orifices) are drilled in a hydrated in-vitro 0.7 mm thick human nail using a Er:YAG laser. The diameter of the orifices ranges from 1 mm to 140 microns.

### Example 2

A series of orifices (ca. 20 orifices) are drilled in vivo without anesthetics in various nails from a healthy subject using a Er:YAG laser. The diameter of the orifices ranges from 1 mm to 50 microns.

### Example 3

A composition containing 10% terbinafine based on the total weight of the composition is prepared and administered to a laser-perforated nail of a patient suffering from onychomycosis once daily for 12 weeks. The progress of clinical effectiveness is seen with growth of the healthy nail.

## Claims

1. Use of a pharmaceutical composition comprising an antifungal for the manufacture of a medicament for use in the treatment of onychomycosis by administration through one or more orifices formed in a nail of a human or animal by a device comprising an Erbium (Er: YAG) laser or Holmium (Ho: YAG) laser.

2. The use of claim 1 wherein the pharmaceutical composition is in a liquid, viscous or semi-solid form.

3. The use of claim 1 or 2 wherein the amount of the antifungal is from 1 to 10% by weight of the composition.

4. The use of any one of claims 1 to 3 wherein the amount of the antifungal is from 10 to 100% by weight of the composition.

5. The use of any one of claims 1 to 4 wherein the amount of the antifungal is more than about 70%.

6. The use of any one of claims 1 to 5 wherein the pharmaceutical composition comprises substantially pure antifungal powder.

7. The use of any one of claims 1 to 6 wherein the pharmaceutical composition comprises a surfactant.

8. The use of any one of claims 1 to 7 wherein the pharmaceutical composition is applied through one or more orifices formed in a nail of a human or animal thereby forming a protective layer onto the nail.

9. The use of any one of claims 1 to 8 wherein the antifungal such as terbinafine is administered to penetrate effectively an orifice of the nail.

10. The use of any one of claims 1 to 9 wherein the antifungal is an allyl amine such as terbinafine or naftifine, a benzylamine such as butenafine, and/or an azole-based antifungal such as tioconazole, econazole, oxiconazole, itroconazole, fluconazole, ketoconazole, miconazole and clotrimazole.

11. The use of any one of claims 1 to 10 wherein the antifungal is terbinafine.

12. The use of any one of claims 1 to 11 wherein the device further comprises supporting means to fix/position the toe or finger to be treated, a pointing laser, and a xyz translation stage module.

13. A device comprising
(a) a laser for perforating nail,
(b) supporting means to fix/position the toe or finger to be treated,
(c) a pointing laser, and
(d) a xyz translation stage module

14. A device according to claim 13, wherein the perforating laser is an Erbium (Er: YAG) or Holmium (Ho: YAG) laser.

15. A device according to claim 13 or 14, wherein the pointing laser is a visible continuous wave (cw) laser e.g. a red He-Ne laser or a laser diode.

16. A device according to any one of claims 13 to 15 further comprising means for monitoring the nail plate.

17. Use of a pharmaceutical composition comprising an antifurigal for the manufacture of a medicament for use in the treatment of onychomycosis by administration through one or more orifices formed in a nail of a human or animal by a device according to any one of claims 13 to 16.

## Patentansprüche

1. Verwendung einer pharmazeutischen Zusammensetzung, umfassend ein Antipilzmittel zur Herstellung eines Arzneimittels zur Verwendung bei der Behandlung von Onychomykose durch Verabreichung durch ein oder mehr Öffnungen, die in einem Nagel eines Menschen oder Tiers gebildet sind, durch eine Vorrichtung, umfassend einen Erbium (Er: YAG) Laser oder Holmium (Ho: YAG) Laser.

2. Verwendung nach Anspruch 1, wobei die pharmazeutische Zusammensetzung in einer flüssigen, viskosen oder halbfesten Form vorliegt.

3. Verwendung nach Anspruch 1 oder 2, wobei die Menge des Antipilzmittels von 1 bis 10 Gew.-% der Zusammensetzung beträgt.

4. Verwendung nach einem der Ansprüche 1 bis 3, wobei die Menge des Antipilzmittels von 10 bis 100 Gew.-% der Zusammensetzung beträgt.

5. Verwendung nach einem der Ansprüche 1 bis 4, wobei die Menge des Antipilzmittels mehr als etwa 70 % beträgt.

6. Verwendung nach einem der Ansprüche 1 bis 5, wobei die pharmazeutische Zusammensetzung im Wesentlichen reines Antipilzmittelpulver umfasst.

7. Verwendung nach einem der Ansprüche 1 bis 6, wobei die pharmazeutische Zusammensetzung ein Tensid umfasst.

8. Verwendung nach einem der Ansprüche 1 bis 7, wobei die pharmazeutische Zusammensetzung angewendet wird durch ein oder mehr Öffnungen, die in einem Nagel eines Menschen oder Tiers gebildet werden, um **dadurch** eine Schutzschicht auf dem Nagel zu bilden.

9. Verwendung nach einem der Ansprüche 1 bis 8, wobei das Antipilzmittel, wie Terbinafin, verabreicht wird, um wirksam durch eine Öffnung des Nagels hindurch zu treten.

10. Verwendung nach einem der Ansprüche 1 bis 9, wobei das Antipilzmittel für ein Allylamin steht, wie Terbinafin oder Naftifin, für ein Benzylamin, wie Butenafin, und/oder für ein auf Azol basierendes Antipilzmittel, wie Tioconazol, Econazol, Oxiconazol, Itroconazol, Fluconazol, Ketoconazol, Miconazol und Clotrimazol.

11. Verwendung nach einem der Ansprüche 1 bis 10, wobei das Antipilzmittel für Terbinafin steht.

12. Verwendung nach einem der Ansprüche 1 bis 11, wobei die Vorrichtung ferner eine Trägereinrichtung umfasst, um den Zeh oder Finger, der behandelt wird, zu fixieren/positionieren, einen Laser-Pointer und ein xyz Translationsbühnenmodul.

13. Vorrichtung, umfassend
(a) einen Laser zur Perforation eines Nagels,
(b) eine Trägereinrichtung, um den Zeh oder Finger, der behandelt wird, zu fixieren/positionieren,
(c) einen Laser-Pointer, und
(d) ein xyz Translationsbühnenmodul.

14. Vorrichtung nach Anspruch 13, wobei der Perforationslaser für einen Erbium (Er: YAG) oder Holmium (Ho: YAG) Laser steht.

15. Vorrichtung nach Anspruch 13 oder 14, wobei der Laser-Pointer ein sichtbarer Dauerstrich (cw) Laser ist, z.B. ein roter He-Ne Laser oder eine Laserdiode.

16. Vorrichtung nach einem der Ansprüche 13 bis 15, die ferner eine Einrichtung zur Überwachung der Nagelplatte umfasst.

17. Verwendung einer pharmazeutischen Zusammensetzung, umfassend ein Antipilzmittel zur Herstellung eines Medikaments zur Verwendung bei der Behandlung von Onychomykose durch Verabreichen durch ein oder mehr Öffnungen, die in einem Nagel eines Menschen oder Tiers gebildet werden, durch eine Vorrichtung gemäß einem der Ansprüche 13 bis 16.

## Revendications

1. Utilisation d'une composition pharmaceutique comprenant un agent antifongique pour la fabrication d'un médicament à utiliser dans le traitement d'une onychomycose par administration à travers un ou plusieurs orifices formés dans un ongle d'un être humain ou d'un animal, par un dispositif comprenant un laser à l'Erbium (Er : YAG) ou un laser à l'Holmium (Ho : YAG).

2. Utilisation selon la revendication 1, dans laquelle la composition pharmaceutique est sous forme liquide, visqueuse ou semi-solide.

3. Utilisation selon la revendication 1 ou 2, dans laquelle la quantité de l'agent antifongique est de 1 à 10 % en poids de la composition.

4. Utilisation selon l'une quelconque des revendications 1 à 3, dans laquelle la quantité de l'agent antifongique est de 10 à 100 % en poids de la composition.

5. Utilisation selon l'une quelconque des revendications 1 à 4, dans laquelle la quantité de l'agent antifongique est supérieure à environ 70 %.

6. Utilisation selon l'une quelconque des revendications 1 à 5, dans laquelle la composition pharmaceutique comprend une poudre antifongique sensiblement pure.

7. Utilisation selon l'une quelconque des revendications 1 à 6, dans laquelle la composition pharmaceutique comprend un agent tensioactif.

8. Utilisation selon l'une quelconque des revendications 1 à 7, dans laquelle la composition pharmaceutique est appliquée à travers un ou plusieurs orifices formés dans un ongle d'un être humain ou d'un animal, formant ainsi une couche protectrice de l'ongle.

9. Utilisation selon l'une quelconque des revendications 1 à 8, dans laquelle un agent antifongique, tel que la terbinafine, est administré pour pénétrer efficacement dans un orifice de l'ongle.

10. Utilisation selon l'une quelconque des revendications 1 à 9, dans laquelle l'agent antifongique est une allylamine telle que la terbinafine ou la naftifine, une benzylamine telle que la buténafine, et/ou un agent antifongique à base d'azole tel que le tioconazole, l'éconazole, l'oxiconazole, l'itroconazole, le fluconazole, le kétoconazole, le miconazole et le clotrimazole.

11. Utilisation selon l'une quelconque des revendications 1 à 10, dans laquelle l'agent antifongique est la terbinafine.

12. Utilisation selon l'une quelconque des revendications 1 à 11, dans laquelle le dispositif comprend en outre des moyens de support pour fixer/positionner l'orteil ou le doigt à traiter, un laser de pointage, et un module de platine de déplacement par translation xyz.

13. Dispositif comprenant :
(a) un laser pour perforer un ongle ;
(b) des moyens de support pour fixer/positionner l'orteil ou le doigt à traiter ;
(c) un laser de pointage ; et
(d) un module de platine de déplacement par translation xyz.

14. Dispositif selon la revendication 13, dans lequel le laser de perforation est un laser à l'Erbium (Er : YAG) ou à l'Holmium (Ho : YAG).

15. Dispositif selon la revendication 13 ou 14, dans lequel le laser de pointage est un laser à onde continue (cw) visible, par exemple un laser rouge He-Ne ou une diode laser.

16. Dispositif selon l'une quelconque des revendications 13 à 15, comprenant en outre des moyens pour surveiller la plaquette unguéale.

17. Utilisation d'une composition pharmaceutique comprenant un agent antifongique pour la préparation d'un médicament à utiliser dans le traitement d'une onychomycose par administration à travers un ou plusieurs orifices formés dans un ongle d'un être humain ou d'un animal, par un dispositif selon l'une quelconque des revendications 13 à 16.
